# EUROPEAN PATENT SPECIFICATION

(11) **EP 2 079 418 B1**
(45) Date of publication and mention of the grant of the patent: **16.07.2014**
(21) Application number: 07870865.8
(22) Date of filing: 09.11.2007
(51) Int. Cl.: A61F 13/00, A61M 1/00, A61L 15/42, A61L 15/64

(54) **POROUS BIORESORBABLE DRESSING CONFORMABLE TO A WOUND AND METHODS OF MAKING SAME**
IN DER FORM AN EINE WUNDE ANPASSBARER PORÖSER UND BIOLOGISCH ABBAUBARER VERBAND SOWIE VERFAHREN ZU SEINER HERSTELLUNG
PANSEMENT BIORÉSORBABLE POREUX POUVANT PRENDRE LA FORME D'UNE PLAIE ET PROCÉDÉS DE FABRICATION DE CE PANSEMENT

(30) Priority: 09.11.2006 US 857814 P; 09.11.2006 US 857903 P; 09.11.2006 US 857902 P
(43) Date of publication of application: 22.07.2009
(73) Proprietor: KCI Licensing, Inc., San Antonio, TX 78265-9508 (US)
(72) Inventor: AMBROSIO, Archel, San Antonio, TX 78249 (US); PAYNE, Joanna, San Antonio, TX 78249 (US); KIESWETTER, Kristine, San Antonio, TX 78210 (US)
(74) Representative: Cordina, Kevin John
(86) International application number: PCT/US2007/023667
(87) International publication number: WO 2008/057600

(56) References cited:
- WO-A1-2004/073697
- WO-A2-2008/129318
- US-A- 5 891 568
- US-A1- 2002 142 992

## Description

### BACKGROUND OF THE INVENTION

### 1. Field of the Invention

The present invention relates generally to methods, systems and compositions for making and using porous bioresorbable dressing in various configurations.

### 2. Description of Related Art

Wound healing may be broadly split into three overlapping basic phases: inflammation, proliferation, and maturation. The inflammatory phase is characterized by hemostasis and inflammation. The next phase consists mainly of epithelialization, angiogenesis, granulation tissue formation, and collagen deposition. The final phase includes maturation and remodeling. The complexity of the three step wound healing process is augmented by the influence of local factors such as ischemia, edema, and infection, and systemic factors such as diabetes, age, hypothyroidism, malnutrition, and obesity. The rate limiting step of wound healing, however, is often angiogenesis. Wound angiogenesis is marked by endothelial cell migration and capillary formation where the sprouting of capillaries into the wound bed is critical to support the regenerating tissue. The granulation phase and tissue deposition require nutrients supplied by the capillaries. Impairments in wound angiogenesis therefore may lead to chronic problem wounds.

Expression of the angiogenic phenotype is a complex process that requires a number of cellular and molecular events to occur in sequential steps. Some of these activities include endothelial cell proliferation, degradation of surrounding basement membrane, migration of endothelial cells through the connective tissue stroma, formation of tube-like structures, and maturation of endothelial-lined tubes into new blood vessels. Angiogenesis is controlled by positive and reduced regulators. In addition to endothelial cells, cells associated with tissue repair, such as platelets, monocytes, and macrophages, release angiogenic growth factors, such as vascular endothelial growth factor (VEGF) into injured sites that initiate angiogenesis.

There are currently several methods used to augment wound healing, including irrigating the wound to remove of toxins and bacteria, local and systemic antibiotics and anesthetics, and local application of growth factors. One of the most successful ways to promote wound healing in soft tissue wounds that are slow to heal or non-healing is reduced pressure therapy. Reduced pressure therapy generally refers to application of a pressure less than the ambient pressure at the wound site, where the magnitude and time period of the reduced pressure treatment is sufficient to promote healing. Examples of devices used to apply reduced pressure include those popularized by Kinetic Concepts, Inc. of San Antonio, Texas, by its commercially available VACUUM ASSISTED CLOSURE® or V.A.C.® product line. The reduced pressure induced healing process has been described in U.S. Patent Nos. 5,636,643 and 5,645,081.

The reduced pressure serves to promote the migration of epithelial tissue and subcutaneous tissue from the healthy tissue towards the wound site. Typical reduced pressure therapy includes application of reduced pressure to a wound site through a dressing that serves as a manifold to distribute the reduced pressure. The dressing is sized to fit the existing wound, placed in contact with the wound, and then periodically replaced with smaller pieces of dressing as the wound begins to heal and becomes smaller. While use of reduced pressure therapy with the dressing has been highly successful, there still exists various difficulties with this process. For example, it may be difficult to obtain a dressing of a proper width, length or depth to properly fit the wound. Further, as the dressing is removed it may also remove healthy tissue, thereby causing further trauma to the wound site.

It has been proposed to use biodegradable materials to make the dressing, thereby resulting in a dressing that need not be removed from the wound site. With many of these dressings, however, the biodegradable polymer is formed in advance into a particular shape. Individual wounds, however, are of inconsistent shapes and sizes.

A need exists, therefore, for a dressing that be easily manufactured and configured to a shape and size to fit the individual patient's wound. A need also exists for a dressing that need not be removed from the wound site. Further, a need exists for a dressing that contains pores such that the dressing can promote healing and healthy tissue growth at the wound site.

### BRIEF SUMMARY OF THE INVENTION

These and other needs are met through the use of bioresorbable dressing containing open cell pores where the dressing is designed to readily conform to the size and shape of the wound site. Thus, in its broadest sense, the invention produces methods, systems and compositions for making and using porous bioresorbable dressing in various configurations. One embodiment in accordance with the invention is the use of one or more bioresorbable polymers, a porogen system and a solvent in the manufacture of a dressing for use in a method for promoting new tissue growth and/or wound healing at a wound site. The dressing is formed by dissolving said one or more bioresorbable polymers and said porogen system in said solvent, and removing said solvent. Said method comprises the steps of: positioning the dressing into the wound site such that the dressing fills the size and shape of the wound site; positioning a manifold in contact with the dressing; covering the manifold with a drape; securing the drape to the skin surface about the wound circumference; applying a reduced pressure to the wound site through the dressing and manifold; and forming pores within the dressing in situ by wound fluids contacting the porogen system within the dressing.

Another embodiment in accordance with the invention is the use of one or more bioresorbable polymers, a solvent and a porogen system in the manufacture of a dressing for use in a method for promoting new tissue growth and/or wound healing at a wound site. The dressing is formed by dissolving said one or more bioresorbable polymers in said solvent, mixing said porogen system with said one or more bioresorbable polymers in said solvent, and removing said solvent. The method comprises the steps of: contacting the dressing with a fluid such that the porogen system forms pores; positioning the dressing into the wound site such that the dressing contacts the wound site; positioning a manifold in contact with the dressing; covering the manifold with a drape; securing the drape to the skin surface about the wound circumference; and applying a reduced pressure to the wound site through the dressing and manifold.

Another embodiment in accordance with the invention is a dressing for use in a method for promoting forming a dressing to be used to support new tissue growth and/or wound healing at a wound site, wherein the dressing is formed by a said method comprising: dissolving one or more bioresorbable polymers in a solvent; adding porogen system particles to said solvent; removing the solvent to form a solid dressing; heat pressing the dressing; and initiating formation of pores by contacting the dressing with a fluid. mixture will precipitate out of solution as one dressing. The residual non-solvent is removed. The resulting dressing is placed into the wound site by being hand molded to the shape and size of the wound. Alternatively, the resulting dressing may be shaped into a rope that is then coiled into the wound to fit the shape and size of the wound. The wound fluids react with the porogen within the dressing, creating pores within the dressing *in situ.* A drape for sealing the dressing is placed over the dressing at the wound site. A reduced pressure delivery tube is fluidly connected to the dressing to delivery a reduced pressure to the wound site.

Another embodiment in accordance with the invention is a method and apparatus for making a porous bioresorbable dressing to be used at a wound site undergoing reduced pressure therapy. In this embodiment, a bioresorbable polymer and plasticizer are dissolved in an appropriate solvent and mixed with a porogen. The resulting mixture is then placed in a non-solvent. The non-solvent should be one that results in the polymer, plasticizer and the porogen precipitating out of solution. The residual non-solvent is removed. The resulting precipitant, *i.e.,* dressing, is placed in a fluid, whereby the fluid reacts with the porogen in the dressing, creating pores within the dressing. The resulting dressing is then dried and placed into the wound by being hand molded to the shape and size of the wound site. Alternatively, the resulting dressing may be shaped into a rope which is then coiled into or onto the wound to fit the shape and size of the wound site. A drape for sealing the dressing is placed over the dressing at the wound site. A reduced pressure delivery tube is fluidly connected to the dressing to deliver a reduced pressure to the wound site.

One embodiment in accordance with the invention is a method and apparatus for making a porous bioresorbable dressing to be used at a wound site undergoing reduced pressure therapy where the dressing also contains factors to promote tissue growth and/or healing. In this embodiment, a bioresorbable polymer is dissolved in an appropriate solvent and mixed with stoichiometric amounts of a porogen. Residual solvent is removed. The resulting dressing is then placed into a fluid, whereby the fluid reacts with the porogen in the dressing, creating pores within the dressing. Once the reaction is complete, the dressing is removed from the fluid and allowed to dry. At this time, the resulting porous dressing may be coated with various substances, including but not limited to, cells, growth factors, or other nutrients that promote cell growth and/or healing. The porous dressing is then placed into the wound site by being hand molded to the shape and size of the wound. Alternatively, the resulting dressing may be shaped into a rope that is then coiled into or onto the wound to fit the shape and size of the wound site. A drape for sealing the dressing is placed over the

Other objects, features, and advantages of the present invention will become apparent with reference to the drawings and detailed description that follow.

### BRIEF DESCRIPTION OF THE DRAWINGS

FIG. 1 illustrates a flowchart in accordance with some embodiments of the invention, demonstrating the process of making a bioresorbable polymer with a sodium biocarbonate and acid porogen system and its use in reduced pressure therapy.
FIG. 2 illustrates a flowchart in accordance with some embodiments of the invention, demonstrating the process of making a bioresorbable polymer with a salt porogen system and its use in reduced pressure therapy.
FIG. 3 illustrates a flowchart in accordance with some embodiments of the invention, demonstrating the process of making a porous dressing by use of a bioresorbable polymer and porogen system, and use of the porous dressing in reduced pressure therapy.
FIG. 4 illustrates a flowchart in accordance with some embodiments of the invention, demonstrating the process of making a porous dressing by use of a bioresorbable polymer and porogen system, and use of the porous dressing in reduced pressure therapy.
FIG. 5 illustrates a flowchart in accordance with some embodiments of the invention, demonstrating the process of making a porous dressing in the shape of a rope and use of the porous dressing in reduced pressure therapy.

### DETAILED DESCRIPTION OF THE PREFERRED EMBODIMENT

In the following detailed description of the preferred embodiments, reference is made to the accompanying drawings that form a part hereof, and in which is shown by way of illustration specific preferred embodiments in which the invention may be practiced. These embodiments are described in sufficient detail to enable those skilled in the art to practice the invention, and it is understood that other embodiments may be utilized and that logical structural, mechanical, electrical, and chemical changes may be made. To avoid detail not necessary to enable those skilled in the art to practice the invention, the description may omit certain information known to those skilled in the art. The following detailed description is, therefore, not to be taken in a limiting sense, and the scope of the present invention is defined only by the appended claims.

All embodiments of the invention include use of a bioresorbable dressing to be used in conjunction with reduced pressure therapy for treatment of a wound site. The invention is not necessarily limited by a specific location of the wound site, nor the type of tissue that is the target of reduced pressure therapy. Thus, the wound site treated by the instant invention may be a location upon or within the body in which it is desired to promote growth and/or healing of the tissue.

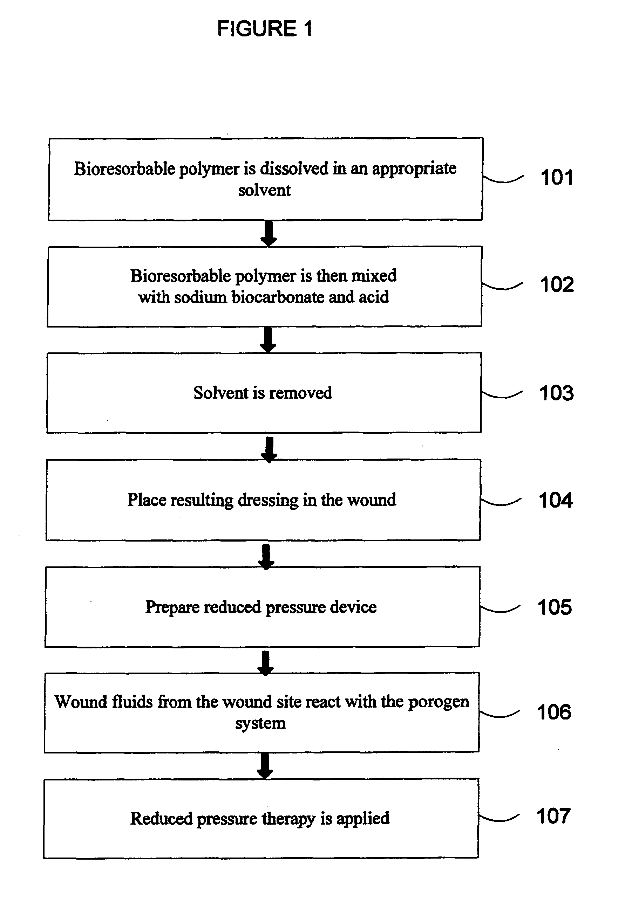

The first embodiment in accordance with the invention is to a method and apparatus for preparing a bioresorbable porous polymer dressing and use of the dressing with reduced pressure therapy, as illustrated in FIG. 1.

To start, one or more bioresorbable polymers is dissolved in an appropriate solvent (101). The type of solvent used will depend upon the bioresorbable polymer(s) selected. The bioresorbable polymer is a biocompatible material whose degradation by products can be bio assimilated or excreted via natural pathways in the body. The bioresorbable polymer may include, but is not limited to, lactide, poly(lactide) (PLA), glycolide polymers, poly(glycolic acid) (PGA), poly(lactide-co-glycolide) (PLGA), ethylene glycol/lactide copolymers, polycaprolactone, polyhydroxybutyrate, polyurethanes, polyphosphazenes, poly(ethylene glycol)-poly(lactide-co-glycolide) co-polymer, polyhydroxyacids, polycarbonates, polyamides, polyanhydrides, polyamino acids, polyortho esters, polyacetals, degradable polycyanoacrylates, polycarbonates, polyfumarates, degradable polyurethanes, proteins such as albumin, collagen, fibrin, synthetic and natural polyamino acids, polysaccharides such as alginate, heparin, and other naturally occurring biodegradable polymers of sugar units. Further, in one preferred embodiment the polymer is a PLA:PCL copolymer, wherein the ratio of PLA to PCL may range from 100:0 to 0:100. In some preferred embodiments, the PLA:PCL copolymer ratio is about 90:10. In other embodiments, the PLA:PCL copolymer ratio is about 80:20. In yet another embodiment, the PLA:PCL copolymer ratio is about 70:30.

A porogen system of sodium biocarbonate and acid is also added to the bioresorbable polymer mixture (102). The acid may be any acid that is not in liquid or gaseous form, thus it is in a solid or crystalline state. Examples of appropriate acids to use therein includes, but is not limited to, citric acid. The amount of sodium bicarbonate and acid used may be used in stoichiometic amounts. It is also envisioned that the sodium bicarbonate may be used in non stoichiometric amounts. Further, the amount of porogen used should be that which creates sufficient number of open cells or channels so that would fluids may be drained and the reduced pressure may continue unimpaired.

The solvent is then removed from the resulting dressing (103). Examples of methods to remove the solvent include, but are not limited to, evaporation, oven drying, vacuum drying, hand kneading and the like. In one embodiment, the solvent is evaporated over a period of about 48 hours.

In one embodiment, the dressing may be heat pressed to compress it and remove any residual bubbles that may exist. The plates of the hot press are preferably covered or coated with a material that inhibits the sticking of the dressing to the plates. Examples of appropriate materials include, for example, Teflon. To increase the porosity of the dressing, the practitioner may cover the top and/or bottom plate(s) of the hot press with additional sodium bicarbonate and acid. In one preferred embodiment, the bottom surface of the dressing is coated with sodium bicarbonate and acid particles with a size of greater than about 500µm and/or the top surface of the dressing is coated with sodium bicarbonate and acid particles with a size of about 90 to about 250µm. Alternatively, one may use an imprinting wafer as the top and/or bottom plate(s) to imprint pores, lines, or other design onto the top and bottom of the dressing. The dressing is heat pressed at a given temperature and pressure, then cooled.

At this stage, the dressing should be malleable. As such, the dressing may be placed in the wound site by use of, for example, hand molding the dressing to fit the shape and size of the wound site (104).

The reduced pressure therapy device is then fluidly connected to the wound site (105). Here, the wound site and the dressing are covered by a drape made of a flexible substance. Preferably, the drape is impermeable, thus blocking or slowing the transmission of either liquids or gas. Preferably, the drape is made of a material that permits the diffusion of water vapor but provides an air-tight seal over the wound site when reduced pressure therapy is applied.. The drape will extend over the surface of the wound site and dressing and extend beyond the edges of the wound. The drape is secured to the skin surface about the wound circumference by, for example, adhesive material. At least one reduced pressure delivery tube is placed beneath the drape, and extends out from underneath the drape. The reduced pressure delivery tube may be made of any medical-grade tubing material, including without limitation, paralyne-coated silicone or urethane. Further, the tubing may be coated with agents to prevent the tubing adhesion to the wound. For example, the tubing may be coated with heparin, anti-coagulants, anti-fibrogens, anti-adherents, anti-thrombinogens or hydrophilic substances. The reduced pressure delivery tube is placed in fluid communication to a reduced pressure source, which preferably comprises a canister safely placed under the vacuum through fluid communication with a reduced pressure source. Thus, in this embodiment, the dressing serves as a manifold to distribute the reduced pressure, assisting in applying reduced pressure to, delivering fluids to, or removing fluids from a wound site.

In an alternate embodiment embodiment, the bioresorbable dressing is placed within the wound site and a manifold is placed over the dressing. The manifold facilitates even reduced pressure distribution over the entire wound site. The wound site, dressing and manifold are then covered by a drape made of impermeable substance that is flexible. The drape will extend over the surface of the wound site, dressing and manifold, extending beyond the edges of the wound and preferably secured to the skin surface. At least one reduced pressure delivery tube is fluidly connected to the manifold. The reduced pressure delivery tube is also placed in fluid communication to a reduced pressure source, which preferably comprises a canister safely placed under the vacuum through fluid communication with a reduced pressure source.

Wound fluids from the wound site then initiate an acid base reaction between the sodium bicarbonate and the acid, producing carbon dioxide gas (106). The carbon dioxide gas production will thus transform the dressing *in situ* into a three-dimensional structure with interconnected pores, or a "scaffold." Further, fluids such as water may be added to the wound site to assist in the porogen system reaction. In an alternative embodiment, step (106) may occur before step (105).

Typically, the pore size produced by the carbon dioxide gas production is about 50 to about 1,500 microns. In one embodiment, the pore size is between about 100 and about 500 microns. In another embodiment, the pore size is between about 100 and about 250 microns. It is understood that the size of the resulting pores is dependent upon the size of the sodium bicarbonate and acid particles and the amount of gas produced. As such, one may use any method to control the size of the sodium bicarbonate and acid particles, including by not limited to, sieving and centrifugation. In one embodiment, the sodium bicarbonate and acid are sieved through one or more screens to produce particles of a certain size. Thus, the pore size will be at a minimum the size of the particles produced by the sieving. If the dressing is malleable enough, the carbon dioxide gas produced will further increase the pore size.

Further, the amount of porogen system used and the particle size of the porogen system will control the percent porosity of the resulting porous dressing. It is understood that the percent porosity preferred by the practitioner may depend upon factors such as the mechanical properties of the materials used within the dressing, such as bioresorbable polymers, the cell infiltration desired, the presence or absence of wound healing or tissue treatment substances, and the like. In one preferred embodiment, the percent porosity is at least about 50%. In another preferred embodiment, the percent porosity is about 70%.

Reduced pressure therapy is then applied to the wound (107). It is understood that the frequency of reduced pressure treatment depends upon the location of the body, the size and shape of the wound site, the exact dressing or dressing used, and the types of various agents applied to the site, if any. Further, depending upon the treatment regiment, reduced pressure therapy may be substantially continuous application or cyclical such that it oscillates the pressure over time. As the wound heals, the porous dressing is resorbed by the body and is replaced by granulating tissue.

In an alternate embodiment, one or more plasticizers is added to the bioresorbable polymer in the solvent (102). Plasticizers may be any material that enhances the deformability of a polymeric compound, adding softening and flexibility to the compound. The plasticizers may include, but are not limited to, cetyl alcohol esters, glycerol, glycerol esters, acetylated glycerides, glycerol monostearate, glyceryl triacetate, glycerol tributyrate, phthalates, dibutyl phthalate, diethyl phthalate, dimethyl phthalate, dioctyl phthalate, citrates, acetyl tributyl citrate, acetyl triethyl citrate, tributyl citrate, triethyl citrate, sebacates, diethyl sebacate, dibutyl sebacate, adipates, azelates, benzoates, vegetable oils, fumarates, diethyl fumarate, malates, diethyl malate, oxalates, diethyl oxalate, succinates, dibutyl succinate, butyrates, cetyl alcohol esters, salicylic acid, triacetin, malonates, diethyl malonate, castor oil, triethylene glycol, and poloxamers. If one or more plasticizers are included in the polymer, then the residual solvent may be removed (103) by any method such as oven drying or vacuum drying as long as the conditions used do not favor evaporation of the plasticizer.

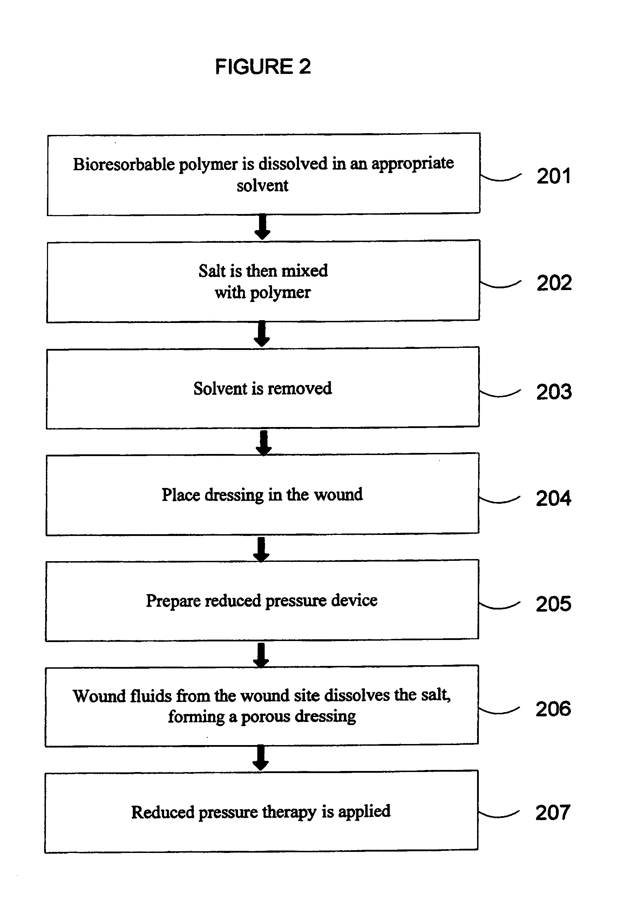

The second embodiment in accordance with the invention is to a method and apparatus for preparing a bioresorbable porous polymer dressing and use of the porous dressing for reduced pressure therapy, as illustrated in FIG. 2.

A bioresorbable polymer is dissolved in an appropriate solvent (201). The bioresorbable polymer may be made of one or more polymers that are bioresorbable. Suitable polymer include those polymers disclosed in other embodiments of the invention. In an alternate embodiment, one or more plasticizers are also added to the bioresorbable polymer.

The bioresorbable polymer is then mixed with a crystalline or solid salt that serves as the porogen system (202). The invention is not limited by the type of salt, as long as the salt is of an appropriate particle size and dissolvable in a fluid, *i.e.,* a gas, liquid, or flowable material, including but not limited to, colloids, dressings, a liquid, a slurry, a suspension, a viscous gel, a paste, a putty, and particulate solids. Examples of appropriate salts used herein includes, but is not limited to, sodium chloride and potassium chloride. The amount of salt used may be used in stoichiometic amounts. It is also envisioned that the salt may not be in non stoichiometric amounts.

The solvent is then removed (203). Examples of methods to remove the solvent include, but are not limited to, evaporation, oven drying, vacuum drying, hand kneading and the like. In one embodiment, the solvent is evaporated over a period of about 48 hours.

In one alternate embodiment, the resulting dressing may be heat pressed to compress the dressing and remove any residual bubbles that may exist. The plates of the hot press are preferably covered or coated with a material that inhibits the sticking of the dressing to the plates. Examples of appropriate materials include, for example, Teflon. To make the dressing later develop into a more porous dressing, the practitioner may cover the top and/or bottom plate(s) of the hot press with additional particles of the salt, *i.e.,* the porogen system. In one preferred embodiment, the bottom surface of the dressing is coated with salt particles of a size of greater than about 500µm and the top surface of the dressing is coated with salt particles with a size of about 90 to about 250µm. Alternatively, one may use an imprinting wafer as the top and/or bottom plate(s) to imprint pores, lines, or other design onto the top and bottom of the dressing. The dressing is pressed at a given temperature and pressure, then cooled.

At this stage, the dressing should be malleable. As such, one may place the dressing in the wound site by use of, for example, hand molding the dressing in the wound site to fill the shape and size of the wound (204).

The reduced pressure device is then fluidly connected to the wound site (205). In this step, the wound site and the dressing are covered by a drape made of impermeable substance that is flexible. Preferably, the drape is made of a material that permits the diffusion of water vapor but provides an air-tight enclosure. The drape will extend over the surface of the wound site and dressing and extend beyond the edges of the wound. The drape is secured to the skin surface about the wound circumference by, for example, adhesive material. At least one reduced pressure delivery tube is placed beneath the drape, and extends out from underneath the drape. The reduced pressure delivery tube may be made of any medical-grade tubing material, including without limitation paralyne-coated silicone or urethane. Further, the tubing may be coated with agents to prevent the tubing adhesion to the wound. For example, the tubing may be coated with heparin, anti-coagulants, anti-fibrogens, anti-adherents, anti-thrombinogens or hydrophilic substances. Thus, in this embodiment, the dressing serves as a manifold to distribute the reduced pressure.

In an alternate embodiment embodiment, the bioresorbable dressing is placed within the wound site and a manifold is placed over the dressing. The manifold facilitates even reduced pressure distribution over the entire wound site. The wound site, dressing and manifold are then covered by a drape made of impermeable substance that is flexible. The drape will extend over the surface of the wound site, dressing and manifold, extending beyond the edges of the wound and preferably secured to the skin surface. At least one reduced pressure delivery tube is fluidly connected to the manifold. The reduced pressure delivery tube is also placed in fluid communication to a reduced pressure source, which preferably comprises a canister safely placed under the vacuum through fluid communication with a reduced pressure source.

Wound fluids, which may include interstitial liquid in the tissues or liquid that has exuded from the tissue or its capillaries of the wound site, will then react with the porogen system, dissolving the salt particles and thus generating pores within the dressing *in situ* (206). Further, fluids such as water may be added to the wound site to assist in the porogen system reaction. The resulting spaces left by the dissolved salt result in a dressing with interconnected pores. The size of the resulting pores is dependent upon the size of the salt particles used. As such, one may use methods to control the size of the salt particles. For example, the salt particles may be sieved through one or more screens to produce particles of a certain size. When the salt particle dissolves, the remaining pore is about the size of the salt particle. The pore size produced by the dissolved salt may be about 50 to about 500 microns. In another embodiment, the pore size is between about 100 and about 400 microns. In another embodiment, the pore size is between about 100 and about 250 microns.

Further, the amount of porogen system used and the particle size of the porogen system will control the percent porosity. It is understood that the percent porosity preferred may depend upon factors such as the mechanical properties of the materials used to make the dressing, the cell infiltration desired, the presence or absence of wound healing or tissue treatment substances trapped within or bound to the dressing, and the like. Wound healing or tissue treatment substances may be covalently or non-covalently bound to dressing by, for example, use of a cross linker, inclusion of a specific reactive group on either the solid support or the s or both molecules, electrostatic interactions, hydrophilic interactions, hydrophobic interactions, attachment by use of molecules such as streptavidin, and use of a combination of covalent and non-covalent interactions.

In one preferred embodiment, the percent porosity is at least about 50%. In another preferred embodiment, the percent porosity is about 70%.

In an alternative embodiment, step (206) may occur before step (205).

Reduced pressure therapy is then applied to the wound (207). As the wound heals, the dressing is resorbed by the body and is replaced by granulating tissue.

In an alternate embodiment, one or more plasticizers is added to the bioresorbable polymer in the solvent (202). If one or more plasticizers are included in the polymer, then the residual solvent may be removed (203) by any method such as oven drying or vacuum drying as long as the conditions used do not favor evaporation of the plasticizer.

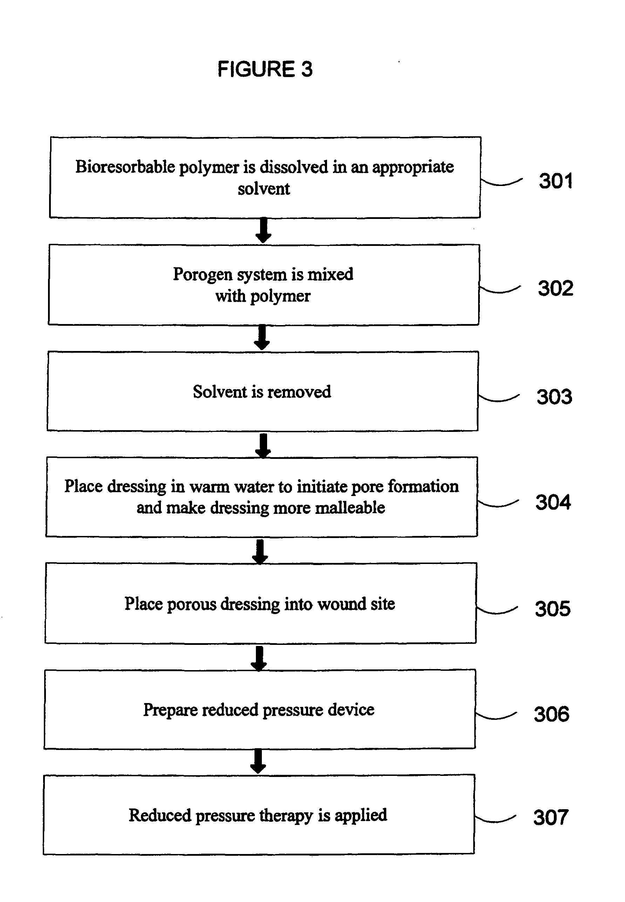

The third embodiment in accordance with the invention is to a method and apparatus for preparing a bioresorbable porous polymer dressing and use of the dressing for reduced pressure therapy, as illustrated in FIG. 3.

One or more bioresorbable polymers are dissolved in an appropriate solvent (301). Suitable bioresorbable polymers may include any polymers discussed in other embodiments of the invention. In one alternative embodiment, at least one plasticizer is also added to the bioresorbable polymer.

The bioresorbable polymer is then mixed with a porogen system, which may include one or more compounds that result in creation of pores within the dressing. (302). The type of a porogen system is not limited, and may include compounds that dissolve when placed in contact with a fluid. This type of porogen system includes inorganic salts like sodium chloride, crystals of saccharose, or gelatin spheres will dissolve in fluids such as water. Another type of porogen system is a mixture of sodium bicarbonate and an acid. Sodium bicarbonate and acid, when placed in contact with a fluid, result in the bicarbonate and acid reacting to form carbon dioxide gas. The gas may then increase the size of the pores.

The solvent is then removed, leaving behind a dressing (303). Examples of methods to remove the solvent include, but are not limited to, evaporation, oven drying, vacuum drying, hand kneading and the like.

In one alternate embodiment, the dressing may be heat pressed to compress the dressing and remove any residual bubbles that may exist. The plates of the hot press are preferably covered or coated with a material that inhibits the sticking of the dressing to the plates, for example, Teflon. To make the dressing later develop into a more porous dressing, the practitioner may cover the top and/or bottom plate(s) of the hot press with additional porogen system particles. In one preferred embodiment, the bottom surface of the dressing is coated with porogen system particles of a size of greater than about 500µm and the top surface of the dressing is coated with porogen system particles with a size of about 90 to about 250µm. Alternatively, one may use an imprinting wafer as the top and/or bottom plate(s) to imprint pores, lines, or other design onto the top and bottom of the dressing. The dressing is pressed at a given temperature and pressure, and then the dressing is cooled

The dressing is then placed in warm water to increase its malleability and react the porogen system, thereby initiating the creation of pores (304). The resulting spaces left by the porogen system result in the dressing being a dressing with interconnected pores. The size of the resulting pores is dependent upon the size of the porogen particles used. As such, one may use means to control the porogen particle size by use of, for example, sieving the particles with screens. Further, the amount of porogen system used and the particle size of the porogen system will control the percent porosity. In one preferred embodiment, the percent porosity is at least about 50%. In another preferred embodiment, the percent porosity is about 70%.

In one alternate embodiment, one or more substances may be used to coat the porous dressing or may be bound to the porous dressing. For example, the dressing may be coated with collagen, hyaluronic acid, gelatin chitosan, antimicrobial agents, therapeutic agents, antiviral agents, growth factors, bioactive substances and other agents that may further facilitate healing and/or tissue growth. Further, the dressing may be coated with a substance to make the dressing radiopaque.

In one embodiment, the dressing is soaked in a solution containing collagen. The dressing is then drained of excess solution and lyophilization of the dressing occurs.

In an alternate embodiment, the dressing is soaked in a solution containing collagen, drained of excess solution and the collagen is then crosslinked onto the dressing.

The dressing is then placed in the wound site by use of hand molding the dressing in the wound site to fill the shape and size of the wound (305). In another embodiment, the dressing is cut to fit the size and shape of the wound. Any wound fluids from the wound site may also act on any remaining porogen system to create further pores.

The reduced pressure device is then fluidly connected to the wound site (306). The wound site and the dressing are covered by a drape made of impermeable substance that is flexible. Preferably, the drape is made of a material that permits the diffusion of water vapor but provides an air-tight enclosure. The drape will extend over the surface of the wound site and dressing and extend beyond the edges of the wound. The drape is secured to the skin surface about the wound circumference by, for example, adhesive material. At least one reduced pressure delivery tube is placed beneath the drape, and extends out from underneath the drape. The reduced pressure delivery tube may be made of any medical-grade tubing material, including without limitation paralyne-coated silicone or urethane. Further, the tubing may be coated with agents to prevent the tubing adhesion to the wound. The reduced pressure delivery tube is also placed in fluid communication to a reduced pressure source, which preferably comprises a canister safely placed under the vacuum through fluid communication with a reduced pressure source. Thus, in this embodiment, the dressing serves as a manifold to distribute the reduced pressure.

In one embodiment, a manifold is placed over the bioresorbable dressing. The manifold facilitates even reduced pressure distribution over the entire wound site. The reduced pressure delivery tube is then fluidly connected to the manifold.

Reduced pressure therapy is then applied to the wound (307). As the wound heals, the dressing is resorbed by the body and is replaced by granulating tissue.

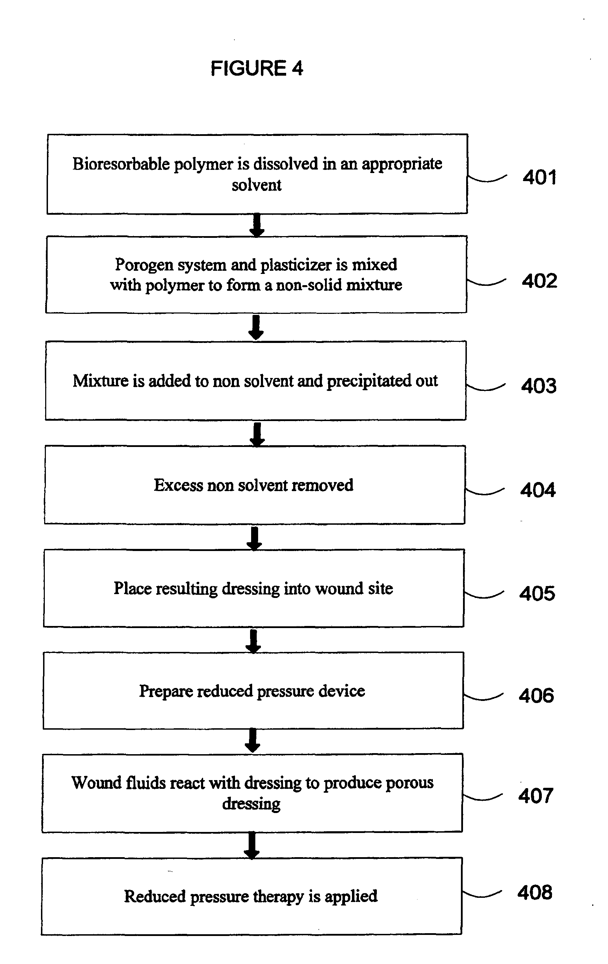

The fourth embodiment in accordance with the invention is to a method and apparatus for preparing a bioresorbable porous polymer and use of the polymer as a dressing for reduced pressure therapy, as illustrated in FIG. 4.

A bioresorbable polymer is dissolved in an appropriate solvent (401). Suitable bioresorbable polymers include, but are not limited to, polymers disclosed in the other embodiments of the invention.

The bioresorbable polymer is then mixed with one or more plasticizers and a porogen system to form a non-solid mixture, such as a fluid or a slurry (402). The porogen system may include but is not limited to, a dissolvable salt or a combination of sodium bicarbonate and acid. The amount of porogen system used may be used in stoichiometic amounts. It is also envisioned that the porogen system may be in non stoichiometric amounts.

The resulting mixture is then added to a non-solvent for the polymer, plasticizer and porogen such that the mixture precipitates out of solution when the mixture comes in contact with the non-solvent (403). Excess non-solvent is then removed from the resulting precipitate (404). Examples of methods to remove the non solvent includes, but is not limited to, evaporation, hand kneading, and the like. Residual solvent may be removed by any method such as oven drying or vacuum driving as long as the conditions used do not favor evaporation of the plasticizer. The resulting dressing may also be heat pressed to remove any residual bubbles that may exist.

The resulting dressing should be malleable. As such, one may place the dressing in the wound site by use of, for example, hand molding the dressing in the wound site to fill the shape and size of the wound (405). Alternatively, the dressing can be molded to fill the shape and size of the wound. In another embodiment, the dressing is shaped by rolling the dressing into a sheet of a desired thickness and cutting the dressing into the desired shape and size of the wound.

The reduced pressure device is then fluidly connected to the wound site (406). In this step, the wound site and the dressing are covered by a drape made of impermeable substance that is flexible. Preferably, the drape is made of a material that permits the diffusion of water vapor but provides an air-tight enclosure. The drape will extend over the surface of the wound site and dressing and extend beyond the edges of the wound, where it is secured to the skin surface by, for example, adhesive material. At least one reduced pressure delivery tube is placed beneath the drape, and extends out from underneath the drape. The reduced pressure delivery tube may be made of any medical-grade tubing material and may be coated with agents to prevent the tubing adhesion to the wound. The reduced pressure delivery tube is also placed in fluid communication to a reduced pressure source, which preferably comprises a canister safely placed under the vacuum through fluid communication with a reduced pressure source. Thus, in this embodiment, the dressing serves to distribute the reduced pressure.

In an alternate embodiment embodiment, the bioresorbable dressing is placed within the wound site and a manifold is placed over the dressing. The manifold facilitates even reduced pressure distribution over the entire wound site. The wound site, dressing and manifold are then covered by a drape made of impermeable substance that is flexible. The drape will extend over the surface of the wound site, dressing and manifold, extending beyond the edges of the wound and preferably secured to the skin surface. At least one reduced pressure delivery tube is fluidly connected to the manifold. The reduced pressure delivery tube is also placed in fluid communication to a reduced pressure source, which preferably comprises a canister safely placed under the vacuum through fluid communication with a reduced pressure source.

Wound fluids from the wound site will then react with the porogen system, forming pores *in situ* (407). The resulting spaces left by the porogen system result in a scaffold, *i.e.,* dressing with interconnected pores. The size of the resulting pores is dependent upon the size of the porogen particles used. As such, one may use means to control the porogen particle size by use of, for example, sieving the particles with screens. Further, the amount of porogen system used and the particle size of the porogen system will control the percent porosity. In one preferred embodiment, the percent porosity is at least about 50%. In another preferred embodiment, the percent porosity is about 70%.

In an alternate embodiment, the porogen system is placed in contact with a fluid before step (406) such that the porogen system reacts with the fluid and forms the interconnected pores before the dressing, i.e., scaffold, is placed in the wound site.

Reduced pressure therapy is then applied to the wound (408). As the wound heals, the dressing is resorbed by the body and is replaced by granulating tissue.

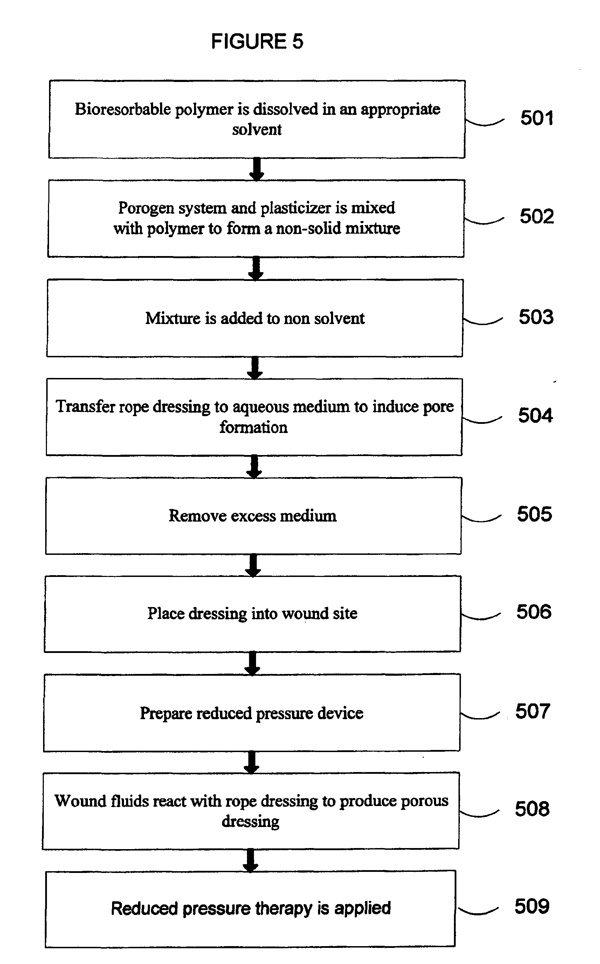

In a fifth embodiment, a porous, bioresorbable rope dressing or dressing is prepared that can be placed into a wound of any size, shape or depth and be able to fill the wound completely because of its rope configuration and flexibility, as illustrated in FIG. 5. The shape of the rope may be non-woven, woven, knitted, braided polymer fibers and the like. These different shapes introduce additional channels or pockets of air, and are based upon the shape of the polymer fibers and the degree of their interwoven nature.

A bioresorbable polymer is dissolved in an appropriate solvent (501). Suitable polymers include, but are not limited to, polymers disclosed in the other embodiments of the invention. The bioresorbable polymer is then mixed with one or more plasticizers and a porogen system to form a non-solid mixture, such as a fluid or slurry (502). The porogen system may include but is not limited to, a dissolvable salt or a combination of sodium bicarbonate and acid. The amount of porogen system used may be used in stoichiometic amounts or non stoichiometric amounts.

The resulting mixture is then extruded through a syringe or other device with a desired tip diameter into a non solvent for the polymer, plasticizer and porogen such that the mixture precipitates out of solution in the form of a string or rope (503). In an alternate embodiment, the rope may be shaped by rolling the resulting mixture into a sheet of a desired thickness and cutting the dressing into a rope shape.

The rope dressing is then transferred to an aqueous medium such as water to react with the porogen system and therefore form a porous dressing (504). The resulting spaces left by the porogen system result in a dressing with interconnected pores. The size of the resulting pores is dependent upon the size of the porogen particles used. As such, one may use means to control the porogen particle size by use of, for example, sieving the particles with screens. Further, the amount of porogen system used and the particle size of the porogen system will control the percent porosity. In one preferred embodiment, the percent porosity is at least about 50%. In another preferred embodiment, the percent porosity is about 70%.

The excess medium is then removed by means including, but not limited to evaporation, hand kneading and the like (505). Further, oven drying or vacuum drying may also be used as long as the conditions used do not favor evaporation of the plasticizer. If necessary, the rope-shaped dressing may also be heat pressed to remove any residual bubbles that may exist.

The resulting dressing should be malleable. As such, the rope-shaped dressing can be coiled into the wound site to fill the shape and size of the wound (506). In another embodiment, two or more ropes are braided or twisted together to form a thicker diameter rope that is then coiled into the wound site.

A reduced pressure device is then fluidly connected to the wound site (507). In this step, the wound site and the dressing are covered by a drape made of impermeable substance that is flexible. Preferably, the drape is made of a material that permits the diffusion of water vapor but provides an air-tight enclosure. The drape will extend over the surface of the wound site and dressing and extend beyond the edges of the wound. The drape is secured to the skin surface about the wound circumference by, for example, adhesive material. At least one reduced pressure delivery tube is placed beneath the drape, and extends out from underneath the drape. The reduced pressure delivery tube may be made of any medical-grade tubing material and may be coated with agents to prevent the tubing adhesion to the wound. The reduced pressure delivery tube is also placed in fluid communication to a reduced pressure source, which preferably comprises a canister safely placed under the vacuum through fluid communication with a reduced pressure source. Thus, in this embodiment, the dressing serves to distribute the reduced pressure.

In an alternate embodiment embodiment, the bioresorbable dressing is placed within the wound site and a manifold is placed over the dressing. The manifold facilitates even reduced pressure distribution over the entire wound site. The wound site, dressing and manifold are then covered by a drape made of impermeable substance that is flexible. The drape will extend over the surface of the wound site, dressing and manifold, extending beyond the edges of the wound and preferably secured to the skin surface. At least one reduced pressure delivery tube is fluidly connected to the manifold. The reduced pressure delivery tube is also placed in fluid communication to a reduced pressure source, which preferably comprises a canister safely placed under the vacuum through fluid communication with a reduced pressure source.

Wound fluids from the wound site react with any residual porogen system, and form additional pores *in situ* (508). Reduced pressure therapy is then applied to the wound (509). As the wound heals, the dressing is resorbed by the body and is replaced by granulating tissue. The rope diameter could vary but preferably would be between about 2 to about 7 mm.

It is also understood that the bioresorbable dressing may be formed by any means suited to the practitioner. For example, in a sixth embodiment, a porous, bioresorbable dressing is prepared by heating one or more bioresorbable polymers above its glass transition temperature such that the polymer is flowable. Suitable polymers include, but are not limited to, polymers disclosed in the other embodiments of the invention. The bioresorbable polymer is then mixed with a porogen system. In another embodiment, one or more plasticizers is also added to the bioresorbable polymer. The resulting mixture is stirred, with or without additional heating, until the biodegradable polymer is mixed with the porogen system. The mixture may then be formed into a sheet or mold and cooled. The resulting mixture may be formed by any means into a shape and size dressing desired to fit the wound site, including but not limited to, hand molding, laser cutting, and the like.

A porous, bioresorbable interfacial layer was prepared by combining a solution of 2.36g of 90:10 PLA:PCL and 0.26g of triethylcitrate in 12 mL of dichloromethane with a mixture of 1.65g citric acid and 2.73g sodium bicarbonate that have been sieved to a particle size or 90-250. The suspension was cast onto a Teflon-coated mold and dried. The resulting sheet was then hot-pressed, soaked in water for 12 hrs to remove the porogen, and dried. V.A.C.^{®} Therapy was simulated using a grid equipped with fluid ports and pressure sensors, a saline infusion rate of 500 mL/day and an applied pressure of 50, 125 or 200 mmHg. The test (n=3) was performed on a 4 x 6 inch sheet of porous, bioresorbable interfacial layer for 48 hours. Fluid was collected and pressure was monitored at pre-determined time points. Tissue ingrowth into the dressing was evaluated using a 5-cm diameter full-thickness excisional swine wound model. The control wounds (n=3) were dressed with a reticulated open-cell dressing while the test wounds (n=3) were covered with porous, bioresorbable interfacial layer with an reticulated open-cell dressing. Continuous V.A.C.^{®} Therapy at -125 mmHg was then initiated. The difference between reticulated open-cell dressing porous, bioresorbable interfacial layer with an reticulated open-cell dressing at each pressure setting was minimal (0.5 - 1.6 mmHg). After 7 days, tissue with dressing was excised en bloc, fixed and H&E stained.

The results indicate that the presence of a porous, bioresorbable interfacial layer under the reticulated open-cell dressing did not impede the fluid flow through the dressing. Ingrowth into reticulated open-cell dressing was extensive when it was placed directly on the wound. Ingrowth into the reticulated open-cell dressing was not observed when porous, bioresorbable interfacial layer was placed between the wound bed. The ingrowth was only seen in the interfacial layer. Thus, removal of just the reticulated open-cell dressing would not have disrupted the new tissue growth if the bioresorbable interfacial layer was used.

It should be apparent from the foregoing that an invention having significant advantages has been provided.

## Claims

1. The use of one or more bioresorbable polymers, a porogen system and a solvent in the manufacture of a dressing for use in a method for promoting new tissue growth and/or wound healing at a wound site, wherein the dressing is formed by dissolving said one or more bioresorbable polymers (101, 201) and said porogen system (102, 202) in said solvent, and removing said solvent (103, 203); and wherein said method comprises the steps of:
positioning the dressing into the wound site such that the dressing fills the size and shape of the wound site (104, 204);
positioning a manifold in contact with the dressing;
covering the manifold with a drape;
securing the drape to the skin surface about the wound circumference;
applying a reduced pressure to the wound site through the dressing and manifold; and
forming pores within the dressing in situ by wound fluids contacting the porogen system within the dressing (106, 207).

2. The use of claim 1, wherein said porogen system is sodium biocarbonate and at least one acid.

3. The use of claim 2, wherein said acid is citric acid.

4. The use of any preceding claim, wherein said porogen system is a salt.

5. The use of any preceding claim, wherein the formation of the dressing further includes the addition of one or more plasticizers to said solvent (102, 202).

6. The use of any preceding claim, wherein the pores are between 100 and 500 microns in size.

7. The use of any preceding claim, wherein positioning the dressing into the wound site occurs by hand molding the dressing.

8. The use of one or more bioresorbable polymers, a solvent and a porogen system in the manufacture of a dressing for use in a method for promoting new tissue growth and/or wound healing at a wound site, wherein said dressing is formed by dissolving said one or more bioresorbable polymers in said solvent (301), mixing said porogen system with said one or more bioresorbable polymers in said solvent (302), and removing said solvent (303), and wherein said method comprises the steps of:
contacting the dressing with a fluid such that the porogen system forms pores (304);
positioning the dressing into the wound site such that the dressing contacts the wound site (305);
positioning a manifold in contact with the dressing;
covering the manifold with a drape;
securing the drape to the skin surface about the wound circumference; and
applying a reduced pressure to the wound site through the dressing and manifold.

9. The use of claim 8, wherein said porogen system is sodium biocarbonate and at least one acid.

10. The use of claim 9, wherein said acid is citric acid.

11. The use of any of claims 8 to 10, wherein said porogen system is a salt.

12. The use of any of claims 8 to 11, wherein the formation of the dressing further includes the addition of one or more plasticizers to said solvent (102).

13. The use of any of claims 8 to 12, wherein positioning the dressing into the wound site occurs by hand molding the dressing.

14. A dressing for use in a method for promoting new tissue growth and/or wound healing at a wound site, wherein the dressing is formed by a method comprising:
dissolving one or more bioresorbable polymers in a solvent (101, 201, 301);
adding porogen system particles to said solvent (102, 202, 302);
removing the solvent to form a solid dressing (103, 203, 303);
heat pressing the dressing; and
initiating formation of pores by contacting the dressing with a fluid (304).

15. The dressing of claim 14, wherein said method further comprises coating the dressing with porogen system particles before heat pressing the dressing.

16. The dressing of claim 14, wherein said method further comprises imprinting a wafer on the top and/or bottom of the dressing by use of heat pressing plates.

17. The dressing of claim 14, wherein said method further comprises: coating the dressing with one or more substances that facilitate healing.

## Patentansprüche

1. Verwendung eines oder mehrerer bioresorbierbarer Polymere, eines Porenbildnersystems und eines Lösemittels bei der Herstellung einer Wundauflage zur Verwendung in einem Verfahren zur Förderung des Wachstums von neuem Gewebe und/oder der Wundheilung an einer Wundstelle, wobei die Wundauflage durch Auflösen des einen oder der mehreren bioresorbierbaren Polymere (101, 201) und des Porenbildnersystems (102, 202) in dem Lösemittel und Entfernen des Lösemittels (103, 203) gebildet wird, und wobei das Verfahren folgende Schritte umfasst:
Positionieren der Wundauflage in die Wundstelle derart, dass die Wundauflage die Größe und Form der Wundstelle (104, 204) ausfüllt,
Positionieren eines Verteilers in Kontakt mit der Wundauflage,
Abdecken des Verteilers mit einer Abdeckung,
Befestigen der Abdeckung an der Hautoberfläche um den Wundrand,
Anlegen eines verringerten Drucks an die Wundstelle über die Wundauflage und den Verteiler und
Bilden von Poren innerhalb der Wundauflage in situ durch Wundfluide, die mit dem Porenbildnersystem innerhalb der Wundauflage in Kontakt kommen (106, 207).

2. Verwendung nach Anspruch 1, wobei das Porenbildnersystem Natriumbiocarbonat und mindestens eine Säure ist.

3. Verwendung nach Anspruch 2, wobei die Säure Zitronensäure ist.

4. Verwendung nach einem der vorhergehenden Ansprüche, wobei das Porenbildnersystem ein Salz ist.

5. Verwendung nach einem der vorhergehenden Ansprüche, wobei die Bildung der Wundauflage ferner das Zusetzen eines oder mehrerer Weichmacher zu dem Lösemittel (102, 202) beinhaltet.

6. Verwendung nach einem der vorhergehenden Ansprüche, wobei die Poren eine Größe zwischen 100 und 500 Mikrometer aufweisen.

7. Verwendung nach einem der vorhergehenden Ansprüche, wobei das Positionieren der Wundauflage in die Wundstelle durch manuelles Formen der Wundauflage erfolgt.

8. Verwendung eines oder mehrerer bioresorbierbarer Polymere, eines Lösemittels und eines Porenbildnersystems bei der Herstellung einer Wundauflage zur Verwendung in einem Verfahren zur Förderung des Wachstums von neuem Gewebe und/oder der Wundheilung an einer Wundstelle, wobei die Wundauflage durch Auflösen des einen oder der mehreren bioresorbierbaren Polymere in dem Lösemittel (301), Mischen des Porenbildnersystems mit dem einen oder den mehreren bioresorbierbaren Polymeren in dem Lösemittel (302) und Entfernen des Lösemittels (303) gebildet wird, und wobei das Verfahren folgende Schritte umfasst:
In-Kontakt-Bringen der Wundauflage mit einem Fluid derart, dass das Porenbildnersystem Poren bildet (304),
Positionieren der Wundauflage in die Wundstelle derart, dass die Wundauflage mit der Wundstelle in Kontakt steht (305),
Positionieren eines Verteilers in Kontakt mit der Wundauflage,
Abdecken des Verteilers mit einer Abdeckung,
Befestigen der Abdeckung an der Hautoberfläche um den Wundrand und
Anlegen eines verringerten Drucks an die Wundstelle über die Wundauflage und den Verteiler.

9. Verwendung nach Anspruch 8, wobei das Porenbildnersystem Natriumbiocarbonat und mindestens eine Säure ist.

10. Verwendung nach Anspruch 9, wobei die Säure Zitronensäure ist.

11. Verwendung nach einem der Ansprüche 8 bis 10, wobei das Porenbildnersystem ein Salz ist.

12. Verwendung nach einem der Ansprüche 8 bis 11, wobei die Bildung der Wundauflage ferner das Zusetzen eines oder mehrerer Weichmacher zu dem Lösemittel beinhaltet (102).

13. Verwendung nach einem der Ansprüche 8 bis 12, wobei das Positionieren der Wundauflage in die Wundstelle durch manuelles Formen der Wundauflage erfolgt.

14. Wundauflage zur Verwendung in einem Verfahren zur Förderung des Wachstums von neuem Gewebe und/oder der Wundheilung an einer Wundstelle, wobei die Wundauflage durch ein Verfahren gebildet wird, das Folgendes umfasst:
Auflösen eines oder mehrerer bioresorbierbarer Polymere in einem Lösemittel (101, 201, 301),
Zusetzen von Porenbildnersystem-Partikeln zu dem Lösemittel (102, 202, 302),
Entfernen des Lösemittels, um eine feste Wundauflage zu bilden (103, 203, 303),
Heißpressen der Wundauflage und
Einleiten der Bildung von Poren durch In-Kontakt-Bringen der Wundauflage mit einem Fluid (304).

15. Wundauflage nach Anspruch 14, wobei das Verfahren ferner das Beschichten der Wundauflage mit Porenbildnersystem-Partikeln vor dem Heißpressen der Wundauflage umfasst.

16. Wundauflage nach Anspruch 14, wobei das Verfahren ferner das Prägen einer Waffelscheibe auf die Ober- und/oder Unterseite der Wundauflage unter Verwendung von Heißpressplatten umfasst.

17. Wundauflage nach Anspruch 14, wobei das Verfahren ferner Folgendes umfasst: Beschichten der Wundauflage mit einer oder mehreren Substanzen, die die Heilung erleichtern.

## Revendications

1. Utilisation d'un ou plusieurs polymères biorésorbables, d'un système porogène et d'un solvant dans la fabrication d'un pansement pour usage dans un procédé de promotion de la croissance d'un nouveau tissu et/ou de la cicatrisation d'une blessure sur un site de blessure, dans laquelle le pansement est formé en dissolvant lesdits un ou plusieurs polymères biorésorbables (101, 201) et ledit système porogène (102, 202) dans ledit solvant et en éliminant ledit solvant (103, 203) ; et dans laquelle ledit procédé comprend les étapes consistant à :
positionner le pansement dans le site de la blessure de sorte que le pansement remplisse la taille et la forme du site de la blessure (104, 204) ;
positionner un collecteur en contact avec le pansement ;
recouvrir le collecteur par un champ stérile ;
fixer le champ stérile à la surface de la peau autour de la circonférence de la blessure ;
appliquer une pression réduite sur le site de la blessure à travers le pansement et le collecteur ; et
former des pores dans le pansement in situ par mise en contact de fluides de la blessure avec le système porogène au sein du pansement (106, 207).

2. Utilisation selon la revendication 1, dans laquelle ledit système porogène est formé de bicarbonate de sodium et d'au moins un acide.

3. Utilisation selon la revendication 2, dans laquelle ledit acide est l'acide citrique.

4. Utilisation selon l'une quelconque des revendications précédentes, dans laquelle ledit système porogène est formé d'un sel.

5. Utilisation selon l'une quelconque des revendications précédentes, dans laquelle la formation du pansement comprend en outre l'addition d'un ou plusieurs plastifiants audit solvant (102, 202).

6. Utilisation selon l'une quelconque des revendications précédentes, dans laquelle les pores ont une taille qui se situe entre 100 et 500 micromètres.

7. Utilisation selon l'une quelconque des revendications précédentes, dans laquelle le positionnement du pansement dans le site de la blessure se fait par moulage à la main du pansement.

8. Utilisation d'un ou plusieurs polymères biorésorbables, d'un solvant et d'un système porogène dans la fabrication d'un pansement pour usage dans un procédé de promotion de la croissance d'un nouveau tissu et/ou de la cicatrisation d'un tissu dans un site de blessure, dans laquelle ledit pansement est formé en dissolvant lesdits un ou plusieurs polymères biorésorbables dans ledit solvant (301), en mélangeant ledit système porogène avec lesdits un ou plusieurs polymères biorésorbables dans ledit solvant (302) et en éliminant ledit solvant (303) ; et dans laquelle ledit procédé comprend les étapes consistant à :
mettre en contact le pansement avec un fluide de sorte que le système porogène forme des pores (304) ;
positionner le pansement dans le site de la blessure de sorte que le pansement vienne en contact avec le site de la blessure (305) ;
positionner un collecteur en contact avec le pansement ;
recouvrir le collecteur par un champ stérile ;
fixer le champ stérile à la surface de la peau autour de la circonférence de la blessure ; et
appliquer une pression réduite au site de la blessure à travers le pansement et le collecteur.

9. Utilisation selon la revendication 8, dans laquelle ledit système porogène est formé de bicarbonate de sodium et d'au moins un acide.

10. Utilisation selon la revendication 9, dans laquelle ledit acide est l'acide citrique.

11. Utilisation selon l'une quelconque des revendications 8 à 10, dans laquelle ledit système porogène est formé d'un sel.

12. Utilisation selon l'une quelconque des revendications 8 à 11, dans laquelle la formation du pansement comprend en outre l'addition d'un ou plusieurs plastifiants audit solvant (102).

13. Utilisation selon l'une quelconque des revendications 8 à 12, dans laquelle le positionnement du pansement dans le site de la blessure se fait par moulage à la main du pansement.

14. Pansement pour utilisation dans un procédé de promotion de la croissance d'un nouveau tissu et/ou de la cicatrisation d'une blessure dans un site de blessure, dans lequel le pansement est formé par un procédé comprenant :
la dissolution d'un ou plusieurs polymères biorésorbables dans un solvant (101, 201, 301) ;
l'addition de particules d'un système porogène audit solvant (102, 202, 302) ;
l'élimination du solvant pour former un pansement solide (103, 203, 303) ;
la compression à chaud du pansement ; et
l'initiation de la formation de pores en mettant en contact le pansement avec un fluide (304).

15. Pansement selon la revendication 14, dans lequel ledit procédé comprend en outre le revêtement du pansement par des particules du système porogène avant compression à la chaleur du pansement.

16. Pansement selon la revendication 14, dans lequel ledit procédé comprend l'enfoncement d'une tranche sur le sommet et/ou sur le fond du pansement par utilisation de plaques de compression à la chaleur.

17. Pansement selon la revendication 14, dans lequel ledit procédé comprend en outre le revêtement du pansement par une ou plusieurs substances qui facilite(nt) la cicatrisation.
